# EUROPEAN PATENT APPLICATION

(11) **EP 3 378 440 A1**
(43) Date of publication of application: **26.09.2018**
(21) Application number: 17162905.8
(22) Date of filing: 24.03.2017
(51) Int. Cl.: A61F 2/14, A61F 9/00, A61F 2/00

(54) **IRIS COVER IMPLANT**

(71) Applicant: KEJAKO SA, 1228 Plan-les-Ouates (CH)
(72) Inventor: ASSOULINE, Michael, 1228 Plan-les-Ouates (CH); ISARD, Pierre-François, 1228 Plan-les-Ouates (CH); BOS, Gilles, 1228 Plan-les-Ouates (CH); ENFRUN, David, 1228 Plan-les-Ouates (CH); MAURER, Aurélien, 1228 Plan-les-Ouates (CH)
(74) Representative: Petit, Maxime

(57) **Abstract**

The invention concerns an iris cover implant (10) intended to cover at least partially the iris of an eye, the cover implant comprising a body having a first face that is an opaque face and, on an opposite second face, at least one attachment member that extends outwardly from the body, the at least one attachment member being able to attach the body to an iris of an eye.

## Description

The invention concerns an iris cover implant.

The young human eye can naturally focus from far to near objects. The mechanism underlying this focus is called the accommodation process. It is composed of three main aspects: first, a convergence of the eye ball as objects get closer, then - for a given luminosity - an increase in the pupil constriction, and finally an adjustment of the crystalline lens shape modulated by the ciliary muscle contraction.

As the eye naturally ages, it becomes harder and harder for it to adjust to near objects. While aging, the subject loses progressively its near vision and is able to create a sharp picture only for far objects. This natural vision affect is called presbyopia. It is assessed that by 2020, around two billion people will have to deal with this optical affect. Presbyopia is experienced as a blurring of the person's vision and frequently arises when the person is reading a document or working at a computer. An untreated patient may compensate by moving the viewed material farther away than would be their previous practice. Roots of presbyopia lays in the aging of the crystalline lens that becomes larger and stiffer, and therefore harder to deform whereas convergence and pupil constriction seem not to be affected.

Many attempts have been done to correct presbyopia. The most common and legacy solution is the use of a pair of reading glasses: as far vision is preserved, the subject can wear glasses to be able to read a book for instance. While this solution is not invasive at all, wearing glasses can be seen as neither aesthetic nor practical for the patient, as he/she needs to put them on and off depending on the distance of the object he is looking at. To solve these problems, multifocal and progressive glasses have been developed. They offer, at least, a first refractive correction for distance viewing and a second refractive correction for viewing near objects; sometimes multiple distance vision by progressive curvature changes.

Surgical attempts have been performed to correct permanently in a more invasive manner the symptoms of presbyopia and restore an amplitude of vision without the need of glasses, often considered as an aging symbol. A common treatment is to induce a static adjustment of the refractive power of one eye of the person. In this way the other eye is used for far vision, and the brain processes the two signals of both eyes to create a new depth of vision that is suitable for near and far images (monovision).

There are different ways to correct an eye for near vision. Changes can be performed on the cornea, by reshaping the latter with laser for instance, or placing a refractive implant inside the corneal tissues (Corneal Inlays). The other solution is to replace the crystalline lens with an implant, adapted to a set of specific distances of vision, named Multifocal Intra Ocular Lens (or MIOLs). This type of device comprises different corrections for a least two specific distances. However this type of treatment suffers drawbacks: it induces a loss of luminosity, halos, glares and flares in night vision for instance.

Original attempts that have been done to try to restore the amplitude of vision may also be noticed such as scleral implants which, for instance, focus on a release of the zonular tension without getting the expected performances.

However, none of the above solutions has proved to be satisfactory.

Taking account of the above, the inventors have searched for a new effective implant to compensate presbyopia.

When searching for such a new implant the inventors have conceived a new implant configuration and have discovered other applications for this new implant configuration.

According to an embodiment of the invention, a new iris implant configuration is an iris cover implant as defined in claim 1.

The iris cover implant is adapted to be located on an iris of a human eye. The iris has a shape of a disc or annulus and the iris cover implant is intended to cover at least a portion of the iris whatever the shape of the implant. Generally speaking, the implant may be located on a radius of the iris and oriented towards the center of the iris.

The iris cover implant may have different shapes, such as an overall shape of a segment, an angular sector or area in some instances etc.

Generally speaking, the iris cover implant body has two opposite faces: a first face is opaque to light and a second one is provided with the attachment member that is intended to cooperate with the iris of the eye (more particularly, the anterior surface of the iris) so as, for the iris cover implant, to be attached to the iris (two functional zones of the body). The opaque face is then intended to be visible through the cornea of the eye. The first and second faces are defined as opposite in that they are faces of the body that are both oriented in opposite directions, one being intended to face the cornea when the implant is attached on the iris, while the other is intended to face the surface of the iris. Both faces may not strictly extend one above the other. One face may even extend further than the other so that the extended portion of this face does not overlap the other one.

Such an iris cover implant is easy to install on the iris (pupillary margin) and efficient.

In addition, it is not intended to be in contact with the iridocorneal angle, which avoids ocular hypertension and following sever complications. It is neither intended to be in contact with the endothelium of the cornea to avoid endothelial failure and following severe complications.

With such a cover implant, more or less severe complications of iris can be avoided: infections, anterior uveitis, ocular hypertension, glaucoma, endothelial failure, corneal oedema and decompensation.

Example of complication secondary to cosmetic artificial iris anterior chamber implants can be found in a case report from Yusrah Shweikh, Sally Ameen and Ali Mearza, BMC Ophthalmology, 201515:97.

The cover implant is intended to be placed into the anterior chamber of the eye, that is, within aqueous humor.

No biocompatibility issue will arise on a long term basis with this new implant configuration. No inflammatory reaction will take place after the installation of such a cover implant.

More generally, such a cover implant may be used for the optical enhancement of an eye, including the compensation of presbyopia, regular ametropia or regular astigmatism.

More particularly, such a cover implant may be used for presbyopia correction or compensation by forming, generally together with other cover implants simultaneously, a diaphragm that is intended to reduce the diameter of the aperture of a constricted iris.

Such a cover implant may alternatively be used for visual or cosmetic enhancement, alteration or repair of the iris anterior surface (visible through the cornea as the "color of the eye").

In such applications a cover implant may be used alone or in combination with other cover implants, e.g. to hide an iris deformity or to locally or wholly modify the color.

In these applications, the iris cover implant or implants are attached on the iris in a position such that the opaque face is above the iris. Here the cover function does not require for the opaque face to extend beyond the iris inner edge.

When searching for a way to compensate presbyopia, the inventors have noted that it could be interesting to reduce the aperture of the eye (pupil) so as to increase the depth of field. In particular, they have noted that the ability of the lens to change its shape decreases with time, but the pupil constriction keeps working. The inventors have also noted that elderly persons who can no longer accommodate with their crystalline lens exacerbate the phenomenon of pupil constriction. Similarly, to the way a photographer increases the depth of field of its camera, pupil constriction leads to a reduction in the diameter of the pupil, thereby suppressing marginal rays. The paraxial rays that remain and pass through the fully constricted pupil are thus able to create a sharp picture on the retina for a wider range of distances of vision. The suppression of these marginal rays using a small aperture is called stenopeic effect or "pinhole effect". However, the inventors have realized that, even though the phenomenon of pupil constriction is exacerbated with elderly persons, this is not sufficient to compensate for the other effects of the eye aging, including presbyopia.

From these statements the inventors have thought of further reducing the diameter of a constricted iris (enhancement of natural pupil constriction) through the use of several iris cover implants, e.g. of the above-mentioned type, to correct presbyopia.

When installed on an iris the plurality of cover implants are arranged in a radial distribution around the central aperture of the iris (pupil) and attached to the iris. The plurality of cover implants may radially converge towards the center of the central aperture.

Each cover implant may be attached on the anterior surface of the iris in a position such that at least one zone of its opaque face that has an optical function forms a cantilevered portion with respect to the inner edge of the iris that bounds outwardly the pupil. Therefore, the opaque optical face extends in the central aperture (the pupil) beyond the iris inner edge when viewed through the cornea. The cantilevered portion with the opaque optical zone may be viewed as a diaphragm portion.

The cover implants may be arranged on the iris so that when pupil is dilated (for far vision and/or in dark conditions) the cover implants are away from each other.

When pupil is constricted (this phenomenon of constriction takes place for near vision and/or under daylight conditions) the cover implants are located one close to another so as to form all together a diaphragm and further reduce the diameter of the natural reduced central aperture of the iris.

By further reducing the aperture of the naturally constricted iris, suppression of the marginal rays can be obtained and the depth of field can be increased (pinhole effect) without significantly and permanently reducing the light adaptation ability of the implanted eye (e.g. luminosity in far vision must be preserved).

Such a diaphragm configuration makes it possible to create a stenopeic effect which efficiently improves near vision.

This pinhole effect is obtained only when needed, i.e. the effect is non permanent whatever the position of the pupil while the cover implants are always present on the iris and move with the latter. When pupil is dilated, the cover implants are away from each other and, therefore, do not lead to a pinhole effect. This effect may be more or less progressive when pupil is constricting. The implant may therefore be considered as dynamic since its position in the eye changes with the iris movement and its technical effect is produced dynamically, i.e. during the constriction movement of the iris.

Thanks to this new iris implant configuration the amount of light is not significantly reduced for distance vision or low light conditions (mesopic) vision. This implant makes it possible to adapt the conditions of luminosity and focus. This implant is adapted to the patient's eye specificities and adapts dynamically so that the vision in night or dark conditions is not limited while a pinhole diameter could be perfectly fitted (customized to the eye).

According to other possible features:
- the opaque face extends at least in a plane that is substantially perpendicular to the direction of extension of the at least one attachment member relative to the second face of the body;
- the at least one attachment member comprises at least two portions that are able to move away from each other under the action of an external force in a plane containing the direction of extension of the at least one attachment member relative to the second face of the body, the body being able to elastically bend within the plane when submitted to an external force so as to cause said at least two portions to move away from each other from an initial position, said at least two portions being able to return to their initial position in the absence of any external force;
- the body has at least two receiving portions that are each adapted to receive an instrument or a portion of an instrument for elastically bending the body; each receiving portion may take the shape of a groove that opens out towards the exterior of the body, i.e. in a direction away from the body;
- the body has at least two attachment members spaced apart from each other along a first direction that is substantially perpendicular to the direction of extension of the at least one attachment member, the body having a thickness along the direction of extension of the at least one attachment member relative to the second face of the body, the thickness of the body being reduced in a zone of the body that is located between the two zones where the at least two attachment members are disposed;
- the body comprises a frame provided with the at least one attachment member and a cover member assembled with the frame, the cover member including the opaque face; the two-part body may be easily manufactured; such a configuration makes it possible to customize the cover member, e.g. by selecting appropriate materials, pigmentation or the like, whereas the frame may be common to all the cover implants; when the cover member is entirely opaque it masks the at least one attachment member from the above (according to a view taken through the cornea, facing the iris);
- the frame and the cover member are mechanically engaged with each other; the cover member may be engaged inside the frame or vice versa; the cover may be inserted in a slot defined in the frame;
- the frame has two receiving portions that are each adapted to receive the cover member; such receiving portions may be inwardly oriented relative to the frame and, e.g. may be facing each other; such receiving portions may take the shape of rails or grooves;
- the frame comprises two parallel spaced apart beams with two attachment members located on the two beams respectively;
- the cover member also includes a mechanical portion that is assembled with the frame;
- the mechanical portion is opaque, transparent or pigmented in a way to obtain a specific visual appearance;
- the cover member and the frame are each able to be elastically deformed; the cover member may take the shape of a flexible foil;
- the at least one attachment member is a clamping member; a clamping member makes it possible to clamp a portion of the anterior surface of the iris; several clamping members may be used;
- the body comprises a frame provided with the at least one attachment member and a cover member including the opaque face, the frame and the cover member being made of a single piece; when the cover member is entirely opaque it masks the at least one attachment member from the above (according to a view taken through the cornea, facing the iris);
- the opaque face has at least one zone that is an opaque optical face; this feature corresponds to an iris cover implant that is, inter alia, a diaphragm implant and has an optical function when implanted on the iris of an eye;
- the opaque optical face belongs to an optical portion that extends beyond the frame; here the optical portion is intended to be located above the pupil margin (beyond the iris inner edge) so as to reduce the diameter of the latter when the cover implant is attached to the iris; to be noted that the entire opaque face of the cover member extends both above the frame and beyond the latter, only the zone extending beyond the frame having an optical function (diaphragm); in another embodiment, the opaque optical face constitutes the only opaque zone of the cover member;
- the opaque optical portion and the mechanical portion may form together a single piece, which proves to be easy to manufacture.

The invention also concerns a surgical kit comprising a plurality of cover implants as defined in any of claims 1 to 14. These cover implants are then placed inside the eye, e.g. through a corneal incision, and next attached to the anterior surface of the iris by means of a specifically designed surgical instrument such as an appropriate surgical forceps. The plurality of cover implants forms a multi-component iris (intraocular) implant acting as a diaphragm when attached to an iris of an eye (presbyopia treatment). The plurality of cover implants provide all together a stenopeic effect as described above when all attached to the iris and the iris is constricted enough.

The invention also concerns a surgical kit comprising a plurality of cover implants as defined in any of claims 1 to 14, the plurality of cover implants forming a multi-component iris (intraocular) implant acting as a partial or total artificial iris when attached to an iris of an eye. Here the cover implants are intended to replace at least in part the visual aspect of the anterior surface on an iris. In practice, the cover implants are intended to at least partially cover the natural iris. This makes it possible to replicate, alter or enhance the cosmetic/visual appearance of the natural iris surface.

The invention also concerns an assembly of several cover implants as defined in any of claims 1 to 14, the plurality of cover implants forming a single iris (intraocular) implant acting as a diaphragm when attached to an iris of an eye. This assembly is intended to be put in place as a whole on the iris.

The invention also concerns an assembly of several cover implants as defined in any of claims 1 to 14, the plurality of cover implants forming a single iris (intraocular) implant acting as a partial or total artificial iris when attached to an iris of an eye. This assembly is intended to be put in place as a whole on the iris.

Other features and advantages will emerge in the course of the remainder description, given by way of non-limiting example only, with reference to the following drawings, in which:
- Figures 1 to 3 illustrate a frame of a cover implant according to an embodiment of the invention;
- Figures 4A-C illustrate a cover member of a cover implant according to an embodiment of the invention;
- Figures 5A-C illustrate the principle of the attachment of a cover implant on the iris;
- Figures 6A-C illustrate the assembling operations of the frame of Figures 1-3 with the cover member of Figures 4A-C;
- Figure 7 is a view of a variant embodiment of an attachment member;
- Figures 8A-C illustrate a Monobloc cover implant according to another embodiment of the invention;
- Figures 9A-C illustrate different possible variant embodiments of an attachment member;
- Figures 10A-E illustrate different possible configurations for the opaque optical portion of a cover implant;
- Figures 11A-C are different successive views showing the pupil constriction and the associated arrangement of cover implants attached to the iris for the cover implant of Figure 10A;
- Figures 12A-C are different successive views showing the pupil constriction and the associated arrangement of cover implants attached to the iris for the cover implant of Figures 10 D-E;
- Figures 13A-D are views showing different possible configurations for the opaque optical of several cover implants attached to the iris;
- Figures 14A-D illustrate the main steps of a possible surgical method for implanting a cover implant on an iris;
- Figure 15 represents a cover implant with no optical function according to an embodiment of the invention;
- Figures 16A-D illustrate different views of another cover implant with both optical and covering functions according to an embodiment of the invention;
- Figure 17 represents the cover implant of Figures 16A-D positioned on an iris;
- Figure 18 represents another cover implant with no optical function positioned on an iris according to an embodiment of the invention;
- Figures 19A-C schematically illustrate the installation constraints for a cover implant according to an embodiment of the invention.

In a first embodiment an iris (intraocular) cover implant carries out a cover or overlap function relative to the iris on which it is intended to be fixed. The terms "cover implant" will be used in the remainder of the description whatever the embodiments and the function/application of the implant since the iris will always be covered by at least a portion of the implant.

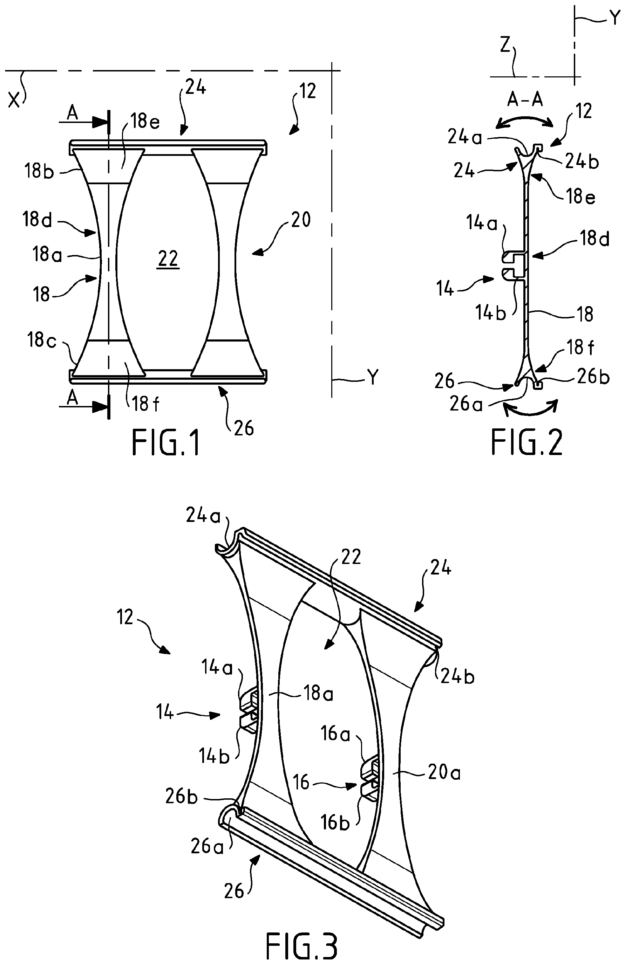

Here the cover implant is made in two parts as illustrated in Figures 1 to 7. The cover implant is made in a biocompatible material or combination or mixture of biocompatible materials. By way of example, the cover implant is made of silicon or hydrophilic acrylics or hydrophobic acrylic or elastomer and, for example, may be cross-linked polydimethylsiloxanes possibly reinforced with silica or EMA (ethyl methacrylate), or HEMA (Hydroxyethyl methacrylate), or a combination of both previous examples (co-polymers) (material adapted for a medical use).

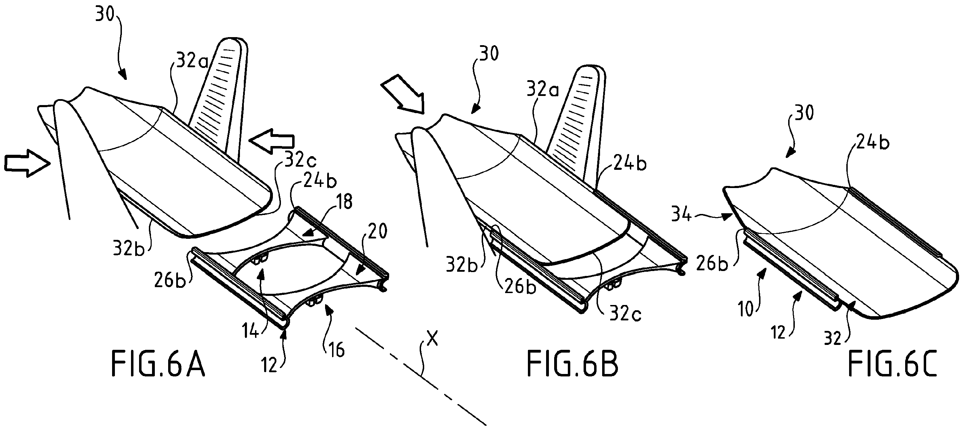

As illustrated in Figures 1 to 3, the cover implant 10 (cover implant 10 is represented in Figure 6C only once assembled) comprises a body that comprises a frame 12 provided with at least one attachment member.

The frame is made of a material or a combination of materials that allow elastic deformation of the frame. PMMA may be an example of appropriate material (transparent or died in the mass).

In the present embodiment there are two attachment members 14, 16 that are disposed on one and the same side or face of the frame that is here oriented downwards.

The frame 12 has an overall axial extension along a longitudinal axis X and transverse extension along a transverse axis Y. The frame 12 also extends perpendicularly to the plane X, Y along axis Z that defines its thickness (fig. 2).

The attachment members 14, 16 extend outwardly from the frame 12 along an overall direction (axis Z) that is substantially perpendicular to the plane XY.

The frame 12 comprises here two transverse beams 18, 20 that are axially spaced apart from each other along axis X so as to leave there between a central opening 22. Cross beams 18, 20 extend along transverse axis Y (fig.1).

The two transverse beams 18, 20 are connected at each of their two opposite ends to two parallel longitudinal or axial supporting members 24, 26.

For instance, the frame is made of a single piece and may be manufactured by an injection molded process or standard machining.

The following description will be made for cross beam 18 for the sake of simplicity and is identical for beam 20.

When viewed in a front or top view (Fig. 1), each transverse beam has a rather narrow central portion (ex 18a for beam 18) which enlarges towards the two opposite ends 18b, 18c where it connects with the supporting members 24, 26 respectively. Each beam has a curved outline in Figure 1 on its two opposite sides. However, other front or top view shapes may be envisaged for the beam.

When viewed in a transverse cross section (AA cross section of Fig. 2), each transverse beam has a rather flat and thin shape in a main portion 18d taken along a direction parallel to axis Z. The main portion 18d includes central portion 18a and a lateral extension on either part thereof. Overall the main portion 18d may extend over a dimension that substantially corresponds here to 2/3 of the length of the beam. However, other ratios may be envisaged. Beyond the main portion 18d the beam comprises enlarged lateral portions 18e and 18f which have an increase in their thickness (taken along axis Z), e.g. a progressive increase. This thickness increase ensures solid mechanical connection with the supporting members 24, 26. Furthermore this thickness increase minimizes deformation, e.g. bending, in the zone of the lateral extensions of the beams. Put it another way, the thickness reduction in the main portion favors deformation, e.g. bending, of the beam, which will contribute to opening of the attachment members.

The thinned configuration of the cross beams in their central and main portions as described above makes it possible to elastically deform the frame in the plane of Figure 2, e.g. by bending as indicated by the two arrows.

In the present embodiment, the two attachment members 14, 16 are located on the underside of the beams 18, 20 respectively in the central portion 18a, 20a thereof.

Each attachment member may be a clamping member and may comprise at least two clamping portions or jaws 14a, 14b and 16a, 16b.

In the present embodiment, the two jaws are spaced apart from each other in their stable, non-deformed initial position as represented in Figures 1 to 3. Depending on the object to be clamped between the two jaws the gap there between may be more or less wide.

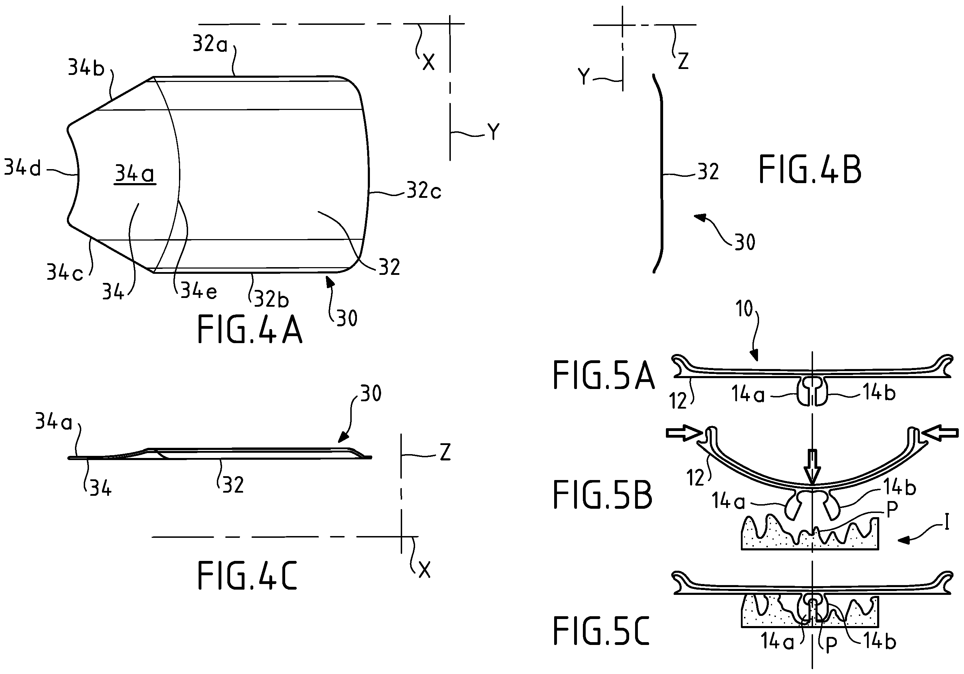

Figure 5A illustrates very schematically the initial position of the clamping jaws 14a, 14b of cover implant 10. In Figures 5A-C cover implant 10 is wholly assembled as in Figure 6C that will be subsequently described. The attachment principle illustrated in Figures 5A-C also applies to a monobloc cover implant.

When an external bending force is exerted on the two supporting members, the frame 12 bends in the plane of Figure 5A (this is also the plane of Figure 2 as indicated by the arrows) and the two jaws of each clamping member move away from each other so as to enlarge the gap (Figure 5B).The cover implant is caused to be moved toward the anterior surface of the iris and the jaws come close to this surface so as to be positioned on either part of an iris ridge.

When the force does no longer exert, the frame returns to its initial position of Figure 5A and the jaws may clamp an iris ridge P (natural iris fold) there between (Figure 5C).

The axial supporting members 24, 26 (Figs 1-3) have each a transverse profile or cross section that may be identical along the whole length or may present locally some controlled irregularities on purpose. Such controlled irregularities may serve as axial stops for a second part of the cover implant body (cover member) when the latter is axially engaged into the frame.

In the present embodiment the axial supporting members 24, 26 may have a first function for accommodating a surgical instrument or part thereof (ex: forceps) that makes it possible to exert an external bending force on the frame to cause its bending and jaws opening as explained above.

In this respect, the axial supporting members 24, 26 may each comprise a first receiving portion 24a, 26a outwardly oriented relative to the frame (Figs 2 and 3).

Here the receiving portion takes the shape of a longitudinal or axial groove opening out to the outside of the frame. The two grooves are opening out on two opposite directions. Each groove has a concave shape which may be adapted to that of the instrument or part thereof in a complementary manner. The opening of the groove may be centered on a plane XY in which the two beams 18, 20 extend. In alternate configurations, the groove may rather be oriented upwardly, i.e. with an opening that makes an angle with the above-mentioned plane.

Further, the axial supporting members 24, 26 may each comprise a second receiving portion 24b, 26b. Both second receiving portions are facing each other and define there between a transverse space. They more particularly define there between together with the upper side or face of transverse beams 18, 20 (the upper side lies in an XY plane) a housing or slot that is adapted to accommodate a cover member.

Second receiving portions 24b, 26b may take the shape of rails or grooves that are inwardly oriented relative to the frame, i.e. substantially towards the beams. Second receiving portions 24b, 26b are more particularly located at a distance from the upper side of the beams (fig 2). In the present embodiment, second receiving portions 24b, 26b are each formed in the same portion of the supporting member as that of the first receiving means. More particularly, each second receiving portion may be formed by one of the two edges that define an adjacent outwardly oriented groove (first receiving portion). This edge is shaped so as to change its orientation towards the inner of the frame and make a bend. Thus the bend defines a concave portion that is inwardly oriented and may accommodate a cover member.

The cover implant body 10 also comprises a cover member 30 (Figures 4A-C) that is intended to be assembled with the frame 12.

The cover member 30 comprises two portions (see Fig.4A which is a top or front view):
- a mechanical portion 32, and
- an opaque optical portion 34.

The two portions form together a single piece in the present embodiment. They may have each the same thickness along axis Z (fig. 2). Here the cover member may take the form of a foil, e.g. a relatively thin foil.

The mechanical portion 32 is to be assembled with the frame 12 thanks to the second receiving portions 24, 26 described above.

The mechanical portion 32 and the opaque optical portion 34 are aligned to each other along the longitudinal axis X. Both mechanical portion 32 and opaque optical portion 34 extend in a plane XY (parallel to the plane of extension of the two beams 18, 20).

Here the cover member visible on Figure 4A has a face (upper face) that has two zones corresponding to portions 32 and 34 respectively. When the cover member is assembled with the frame, this upper face forms a first face that is opposite the face of the frame that carries the attachment members.

The mechanical portion 32 may be transparent to light and maintain the initial aspect of the iris (the corresponding first zone of the upper face of the cover member is therefore not opaque). The mechanical portion 32 may alternatively be pigmented or textured so as to produce a visual effect (ex: pigments as those of an iris). The corresponding first zone of the upper face of the cover member is therefore opaque.

When viewed from above (as in Figure 4A), the mechanical portion 32 has a substantially rectangular shape with two substantially parallel longitudinal edges 32a, 32b, a rear edge 32c with a convex shape.

The opaque optical portion 34 axially extends mechanical portion 32 at its front zone by an appropriate shape that may vary according to the embodiments. The portion 34 has a function of being opaque, i.e. non transparent to light so as to produce a stenopeic or pinhole effect. Here this is the second zone 34a of the upper face of the cover member that is opaque. Generally, the portion 34 may be opaque across its whole thickness (figs. 4A and 4C) that is intended to be oriented in the direction the eye is looking at when the cover implant 10 is installed on the iris of the eye. Thus the opaque optical portion 34 is configured to receive incident light and stop corresponding rays. In a preferred embodiment, portion 34 is a black optical portion. In alternative embodiments other opaque visual appearance for face 34a such as colored, pigmented visual appearance, may be envisaged.

Here the opaque optical portion 34 has two converging edges 34b 34c that extend from longitudinal edges 32a, 32b respectively and terminate at an end edge 32d that extends transversally and connects both inclined edges 34b, 34c.

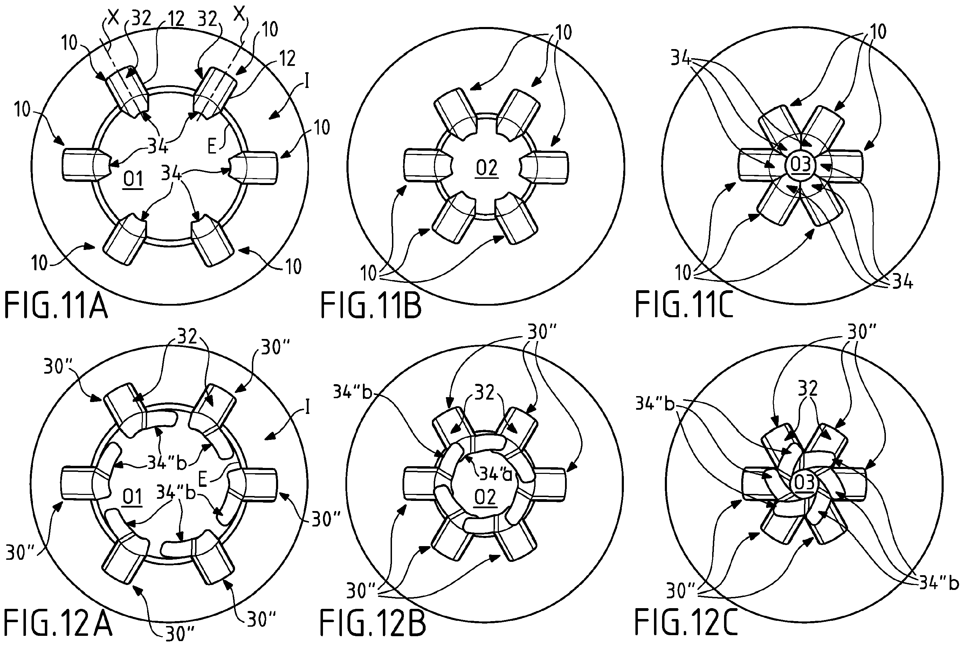

Edges 34b and 34c have a geometry that adapts to the geometry of other opaque optical portion edges of adjacent cover implants when all are installed on the iris of an eye around the pupil and the latter is in its most constricted position as will be seen subsequently. Here the inclination angle of the edges is the same for all the opaque optical portion edges of the cover implants so that two adjacent converging edges are in contact. Thus, no light will be able to pass between two adjacent edges in this embodiment. Figure 11C illustrates this pupil position with all cover implants 10 in close relationship to each other. To be noted that in other cover implants configurations light may pass between adjacent cover implants without adversely affecting the stenopeic effect. This is the case in particular when the gap between adjacent cover implants represents a small negligible portion of the whole diaphragm (e.g. on the order of 3 or 5%).

End edge 34d has here a concave shape in a top view that is able to reproduce as much as possible an overall substantially circular outline when all the cover implants are installed on the iris of an eye and the latter is in the most constricted position of the pupil.

Put it another way, when all the cover implants are in a close relationship (most constricted position of the pupil) adjacent to each other, the opaque optical portions form, and act as, a whole diaphragm that is as homogeneous as possible (continuity in the opacity).

Different other possible shapes of opaque optical portions will be described later on.

As illustrated in Figure 4A, opaque optical portion 34 is bounded by a line 34e that is at the frontier with mechanical portion 32 and separates both portions. Line 34e has substantially the same curvature as edge 34d so as to define there between a portion of an annular shape or annulus. All these opaque portions placed side by side in the most constricted position of the pupil form a continuous opaque annular or ring shape (diaphragm of Fig 11C).

As represented in Figure 4A, the length or axial extension of optical portion 34 is smaller than that of mechanical portion 32 and for instance may be half the length of the latter. By way of example the total length of both portions is 2.22 mm.

The cover member is able to be elastically deformed, i.e. here along its longitudinal axis (parallel to axis X), by bending around this axis.

In a general manner, the cover member 30 overall extends in a plane (XY plane). It is however elastically deformed as represented in Figures 4B and 4C in order to be assembled with the frame as will be seen subsequently. This is because the beams 18, 20 have raised lateral portions (see Fig. 2). Once the cover member has been elastically deformed and assembled with the frame the cover member keeps inside a residual stress that makes it possible to be maintained in position within the frame.

As represented in Figures 4B and 4C, the cover member 30 has a substantially curved shape along its longitudinal axis, e.g. as a tile.

Here cover member 30 is flexible and may be made, e.g. of silicon or hydrophilic acrylics or hydrophobic acrylic or elastomer.

Figures 6A-C illustrate the assembling process of the cover member 30 and frame 12.

In Figure 6A, cover member 30 is held by a forceps in a slightly folded configuration and moved towards the frame along longitudinal axis X with the rear zone (edge 32c) facing the frame. The frame is placed with its second receiving portions 24b, 26b oriented upward and attachment members downward. To be noted that frame 12 has no front and rear parts (this however may be the case for other frame embodiments), which makes the assembling process easier and faster.

Prior to any assembling operation the cover member 30 is bent as explained above (this is done through using conventional forceps as represented in Figure 6A) to take a curved shape which substantially corresponds to the inner frame outline defined by the upper face of the frame (upper face of beams 18, 20) and the raised lateral portions 24b, 26b (ex: the flared inner shape of the lateral supporting members 24, 26) of the frame. In a transverse cross section the inner frame outline has substantially a U shape and defines an open housing for accommodating cover member 30.

The longitudinal edges 32a and 32b are engaged or inserted into second receiving portions 24b 26b above cross beams 18, 20 and then are caused to slide along these portions (ex: in the rails or grooves) as illustrated in Figure 6B.

The cover member 30 is axially pushed until the opaque optical portion 34 substantially reaches the free ends of second receiving portions 24b 26b (Figure 6c). Appropriate local configuration(s) inside the second receiving portions may be provided to axially stop the sliding movement of cover member 30. By way of example, a lug or a slightly conical shape formed by the whole set of the two second receiving portions may be used.

When the cover member has been installed within the frame, these two parts are maintained together through friction and contact pressure.

Thus, opaque optical portion 34 axially extends (along axis X) beyond the frame as a cantilevered beam. This holds true whatever the shape of the cover member, in particular the opaque optical portion. This arrangement makes it possible to attach the cover implant on the iris in such a position that only the opaque optical portion extends beyond the iris edge or pupil margin (see e.g. the relative position between cover implant 10 and iris edge E on Figure 11A).

To be noted that in Figure 6C a rear fraction of the mechanical portion 32 also protrudes from the frame so as to serve as counterweight. In other embodiments the mechanical portion may be flush with the frame.

Other embodiments not depicted here may cover an inverted configuration in which the frame is engaged inside the cover member. The cover member may be designed so as to incorporate receiving portions for accommodating the frame. For example, two receiving portions may extend respectively from two substantially parallel edges of the cover member (as edges 32a and 32b), e.g. in a perpendicular direction to the cover member, and may cover the frame placed below on three or four sides as a cap: the upper side and two downwardly extending lateral sides with a possible angled terminating portion extending under the frame so as to form a lower side. Assembling the cover member and the frame member may be achieved by axially engaging the frame into the lower receiving portions of the cover member.

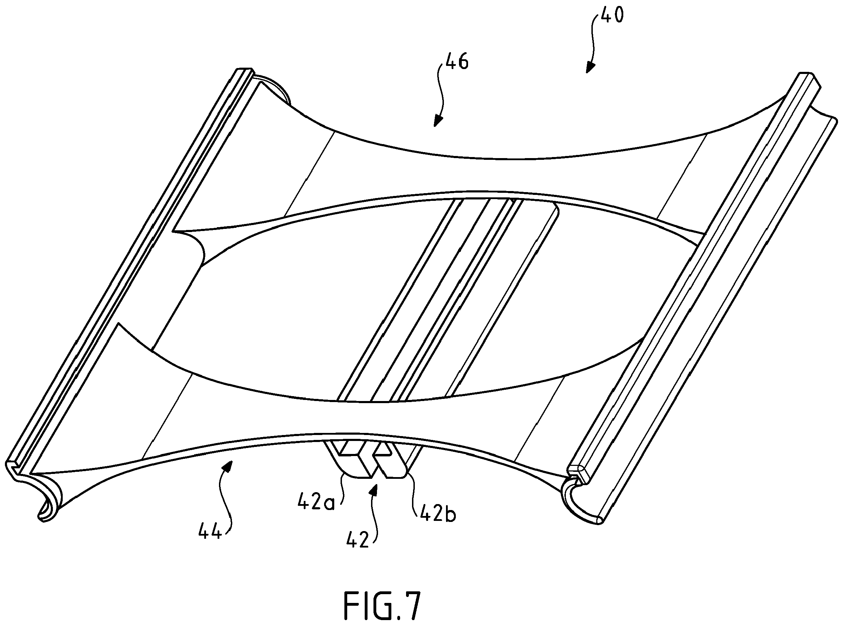

Figure 7 illustrates a variant embodiment of a frame 40 with a single attachment member. Here the attachment member is a clamping member 42 that axially extends from one transverse beam 44 to the other 46.

Clamping member 42 has two axially elongated seizing portions or jaws that behave as jaws 14a, 14b in Figures 5A-C. The jaws are each attached to the lower face of the two beams. In this embodiment all the other components of the frame are identical to those of the previous frames.

To be noted that in other variant embodiments the attachment members may be arranged differently relative to each other, e.g. not necessarily along the same axial direction of the frame (along axis X) but in a laterally offset position.

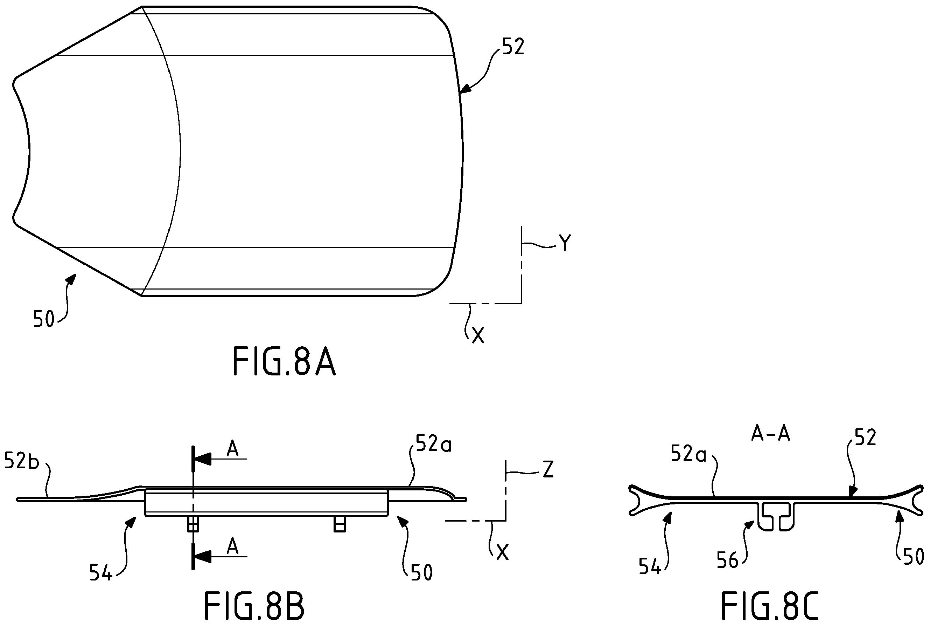

Figures 8A and 8B illustrate an iris cover implant 50 comprising a body that is made as a single piece for easiness of manufacture and implementation by a surgeon. The body comprises a cover member 52 and a frame 54 that are formed as a whole. Here the frame has no second receiving portions. The cover member 52 has a mechanical portion 52a and an opaque optical portion 52b that have the same features and advantages as for the embodiments and variants with a two-part body. Frame 54 also comprises attachment members 56 with the same features and functionalities as for the embodiments and variants with a two-part body. The underside of the frame is recessed between the two attachment members so as to provide flexibility to the frame (not depicted here). All that has been described above also applies to the present embodiment.

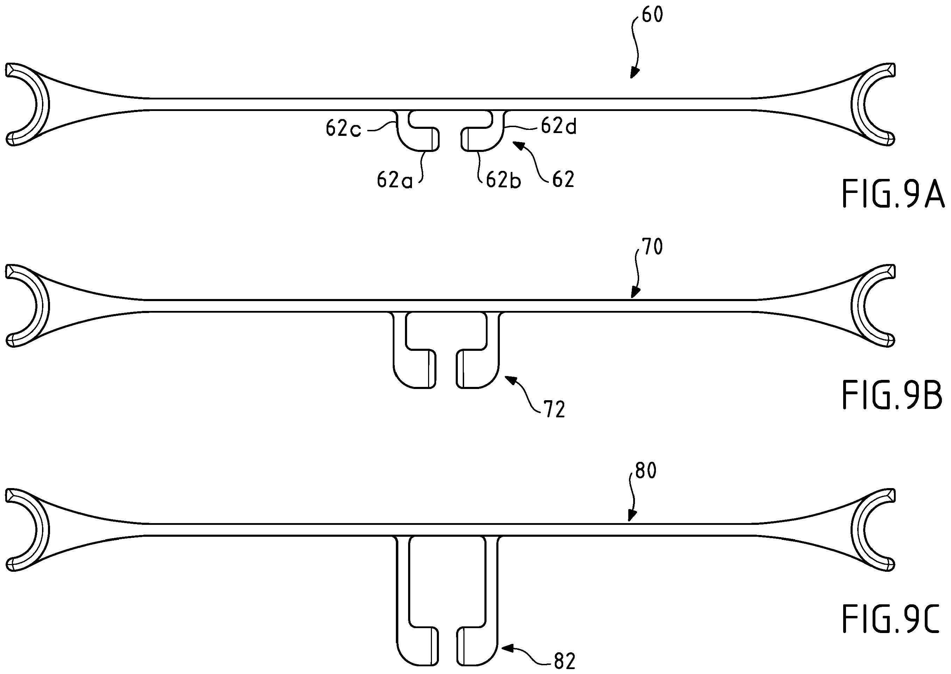

Figures 9A-C show various possible configurations of an attachment member fixed under a frame of an iris cover implant. Figures 9A-C illustrate clamping members with two spaced apart clamping portions or jaws of various sizes.

In Figure 9A, the frame 60 is provided with a clamping member 62 that has two facing heads 62a, 62b linked to the underside of frame 60 through respective downwardly extending arms 62c, 62d.

In Figures 9B and 9C the respective frames 70 and 80 have clamping members 72 and 82 with larger and larger heads and longer and longer arms.

Other possible configurations may be envisaged, e.g. with different head shapes, different number of seizing portions or jaws etc.

As for the previous embodiments the attachment members may be located under the two spaced apart transverse beams in the central portion thereof or may be arranged at another location. As in Figure 7 a single attachment member may be provided under the frame. The frame may also vary in shape.

To be noted that in Figures 9A-C the opposite first receiving portions of each frame have been schematically represented by a symmetrical groove relative to the median plane X, Y of the transverse beams.

In the present embodiments of Figures 9A-C the body of the cover implant (frame and cover member) may be made as a single piece and no second receiving portions are therefore necessary.

However, Figures 9A-C also apply to cover implants the body of which is in two parts as in Figures 1 to 7.

The attachment members of Figures 9A-C may be used in any of the previous embodiments and variants.

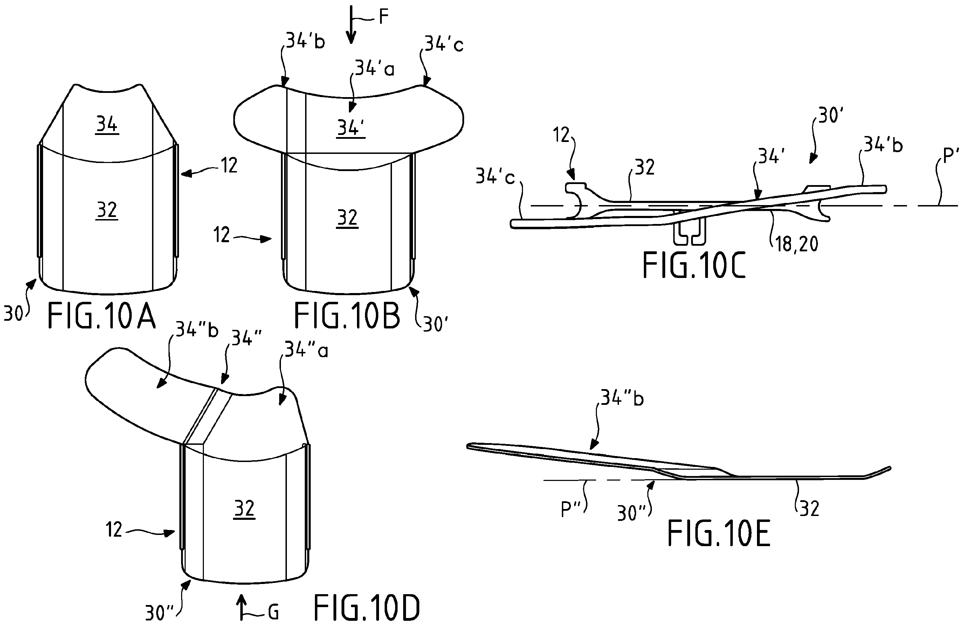

Figures 10A-E illustrate various possible configurations of cover members mounted to a frame 12 including the already described cover member of Figure 10A.

Figures 10B-E show different cover member configurations in which the mechanical portion 32 remains the same as in Figure 10A and the opaque optical portions differ from each other and from opaque optical portion 34.

In Figure 10B the cover member 30' has a substantially T-shape with the opaque optical portion 34' corresponding to the T crossbar.

The opaque optical portion 34' has a substantially curved shape, e.g. a bean shape, with a central curved zone 34'a and two side zones 34'b and 34'c that laterally extend beyond portion 32 and frame 12 in a flared manner viewed from above. Central curved zone 34'a is axially aligned with mechanical portion 32 when viewed from the above.

As for portion 34 in Figure 10A, portion 34' has rounded edges (no sharp angles). Central curved zone 34'a has the same kind of curvatures as portion 34 for the same reasons.

Side zones 34'b and 34'c are not lying in a plane as that of Figure 10B.

Figure 10C represents an elevation view of the whole cover implant taken from the arrow F in Figure 10B (from the end side of the diaphragm where the opaque optical portion 34' is located). In this Figure the side zones 34'b and 34'c are inclined relative to a XY plane P' (here horizontal in Figure 10C) in which the transverse beams 18, 20 substantially extend, at least in their main central portions. In this slanted position the side zone 34'b forms an upper zone that extends above plane P' and side zone 34'c forms a lower zone that extends below plane P'.

This arrangement makes it possible to appropriately dispose adjacent opaque optical portions during the diaphragm closing (when the pupil is being constricted) so that they do not mechanically interfere with other. The adjacent opaque optical portions may thus partially overlap when seen from the above.

Such a configuration allows to provide an efficient homogeneous opaque diaphragm or cover with all the adjacent opaque optical portions of all the cover implants.

In Figure 10D the cover member 30" has not a symmetrical shape when viewed from the above as for cover members 30 and 30'. In contrast, cover member 30" has a substantially L shape with an opaque optical portion 34" extending sideways. Opaque optical portion 34" has a central zone 34"a that is axially aligned with mechanical portion 32 when viewed from the above. Opaque optical portion 34" has also a side zone 34"b that extends laterally in a slanted and forward direction (viewed from the above) relative to the alignment axis of portions 34"a and 32.

Opaque optical portion 34" has a curved end edge 34"c that is substantially concave along its length so as to reproduce a substantially circular outline when all the cover implants are adjacent to each other in the most closed configuration of the whole diaphragm implant (pupil fully constricted).

More particularly, opaque optical portion 34" has a double concavity instead of a single one for opaque optical portions 34 and 34'.

Figure 10E shows a rear view of the cover member 30" (view along the arrow G of Figure 10D) without its frame 12.

As represented in this transverse view, opaque optical portion 34" is not lying in a plane as opaque optical portion 34' but makes an angle with the plane P" (here horizontal plane) in which mechanical portion substantially lies.

This arrangement makes it possible to appropriately dispose adjacent opaque optical portions during the diaphragm closing (when the pupil is being constricted) so that they do not mechanically interfere with other. The adjacent opaque optical portions may thus partially overlap when seen from the above.

Such a configuration allows to provide an efficient continuous opaque diaphragm or cover with all the adjacent opaque optical portions of all the cover implants.

Figures 11A-C show a plurality of iris cover implants 10 implanted (in particular attached) on the anterior surface of a human iris I. When not implanted on the iris, the plurality of cover implants 10 form a surgical kit that is ready for use by the surgeon.

In a variant the surgical kit may comprise separate cover members and separate frames (when the cover implants are not in a single piece) that need to be assembled together prior to any iris implantation by the surgeon.

Figures 11A-C show different positions of the pupil.

In the initial dilated position (fully open) of Figure 11A the cover implants 10 are radially arranged relative to the centre of central aperture O1 with their longitudinal axis (X) radially oriented and far away from each other.

The central aperture O1 bounded by the iris sphincter edge E is at its maximum diameter (ex:7 mm).

The cover implants 10 have a rather small opaque optical portion 34 such that in this dilated position where they are far from each other they may be considered as not affecting the vision.

In Figure 11B the pupil is partially constricted and the central aperture has reduced to 02 (the reduced diameter is e.g. of 4.5 mm).

The cover implants 10 are caused to be moved by the iris movement and get closer to the centre of the aperture and from each other.

In Figure 11C the pupil is fully constricted and the central aperture has reduced to 03 (the reduced diameter is e.g. of 3 mm).

The cover implants 10 come into contact with each other two by two or in very close proximity so that the opaque optical portions 34 form all together a substantially continuous opaque diaphragm or zone (ex: here a band-shaped zone) around reduced aperture 03. This produces a pinhole or stenopeic effect which increases the depth of field.

Here the close contact or proximity between the adjacent cover implants 10 is made possible thanks to the shapes of the opaque optical portions 34 that are adjusted to be positioned side by side in a same plane.

Figures 12A-C show a plurality of the iris cover implants 30" of Figures 10D-E implanted (in particular attached) on the anterior surface of a human iris I as in Figures 11A-C and with the same pupil movements.

In contrast to cover implants 10, cover implants 30" are not lying in a planar configuration due to their upwardly inclined or raised side zones 34"b (see Fig. 10E).

In the dilated position of Figure 12A, the mechanical portions 32 of cover implants 30" are spaced apart from each other whereas the adjacent opaque optical portions 34" are rather close to each other. However, it does not matter since the aperture O1 is large enough.

In Figure 12B the cover implants 30" already overlap each other due to their laterally extending opaque optical portions 34", in particular their raised side zones 34"b. The side zone 34"b of each opaque optical portion 34" partially overlaps central zone 34"a of the adjacent opaque optical portion 34" in the left position. This arrangement creates a substantially continuous opaque annular or ring shape diaphragm around aperture 02 (this therefore starts the pinhole effect process) whereas in corresponding Figure 11b the cover implants 10 are farther from each other (no pinhole effect has been created yet). In Figure 12B, the mechanical portions 32 of the cover implants 30" are at a distance from each other as in Figure 11B.

In Figure 12C the cover implants 30" get closer to each other as the pupil reduces to aperture 03. The cover implants 30" come into contact with each other two by two or in very close proximity by their mechanical portions 32 (as in Figure 11C). The side zones 34"b of the opaque optical portions 34" are no longer laid out along the annular or ring shape of Figure 12B. They are now angularly shifted towards the outside of aperture 03, i.e. oriented outwardly from the annular shape.

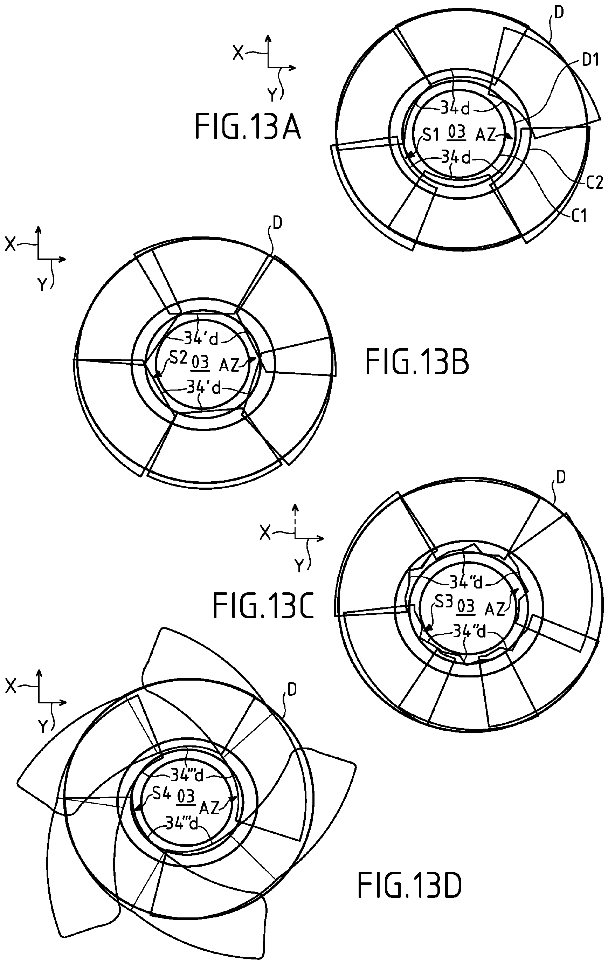

Figures 13A-D show different configurations of opaque optical portions in the most constricted position of the pupil. The diameter of the constricted pupil is denoted by D.

In these Figures the opaque optical portions may overlap (since they have not a planar configuration) two by two not necessarily in a regular manner.

These opaque optical portions have all end or terminating edges that are at least partially facing the central aperture 03 of the pupil when the corresponding cover implants are close to each other.

What matters is that the outline of the central aperture 03 bounded by these end edges be located in an annular zone AZ centered on a diameter or circle D1 that corresponds to the targeted diameter for the diaphragm in the most constricted pupil position so as to obtain the desired stenopeic effect. The zone Z is outwardly bounded by two circular lines C1 and C2 that represent tolerances margins with respect to the target D1.

In Figure 13A the opaque optical portions have a configuration with a concave end edge 34d as in Figure 4A but the opaque optical portions are slanted relative to an XY planar configuration so as to be able to overlap as illustrated in Figure 13A. The opaque optical portions are arranged in such a way to form an outline S1 bounding aperture 03 that is irregular in shape but contained within zone Z.

In Figure 13B the opaque optical portions have a configuration with a rectilinear end edge 34'd and are slanted relative to an XY planar configuration so as to be able to overlap as illustrated in Figure 13B. The opaque optical portions are arranged in such a way to form an outline S2 bounding aperture 03 that is here regular in shape (an hexahedron) and contained within zone Z.

In Figure 13C the opaque optical portions have a configuration in which the end edge 34"d has an uneven relief and the opaque optical portions are slanted relative to an XY planar configuration so as to be able to overlap as illustrated in Figure 13C. The opaque optical portions are arranged in such a way to form an outline S3 bounding aperture 03 that is here irregular in shape and contained within zone Z.

In Figure 13D the opaque optical portions have a configuration in which the end edge 34"'d has a concave shape and the opaque optical portions are slanted relative to an XY planar configuration so as to be able to overlap as illustrated in Figure 13D. The configuration of the opaque optical portions is as that of Figures 10D and E with an inverted shape (the lateral extension is on the right of central zone 34"a). The opaque optical portions are arranged in such a way to form an outline S4 bounding aperture 03 that is here irregular in shape and contained within zone Z.

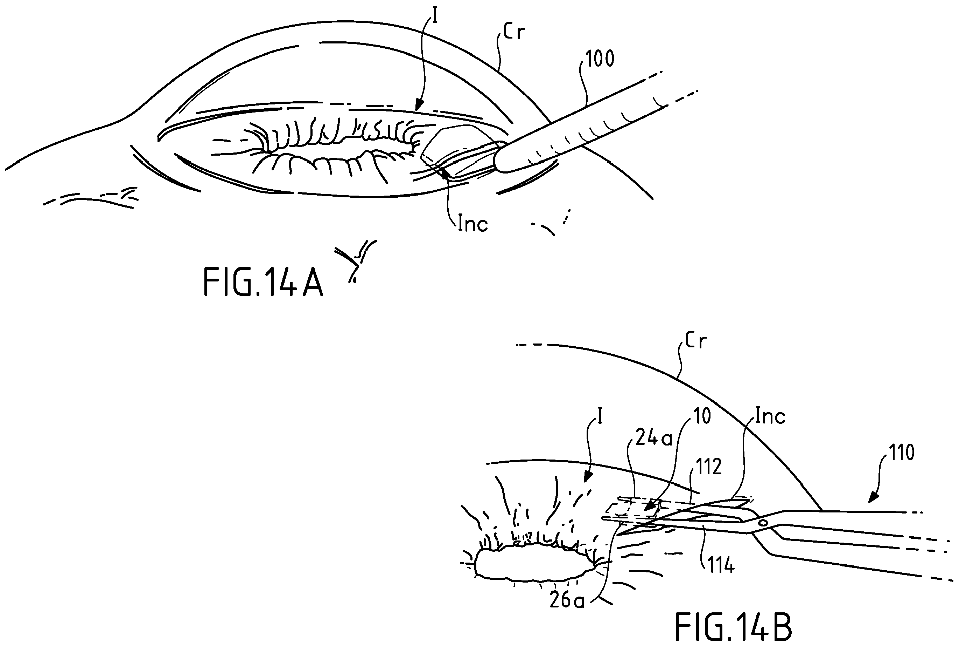

Figures 14A-D illustrate the main steps of a possible surgical method for implanting on the anterior surface of iris I a plurality of iris cover implants as any of the above-described elements, e.g. cover implants 10.

In present embodiment the cover implants may form part of a surgical kit.

In a first step (Fig. 14A), the cornea Cr of the eye is incised e.g. using a conventional surgical instrument, e.g. a surgical blade 100. Alternatively a femtosecond laser may be used to make an incision Inc. the incsision may be a limbal self sealing clear corneal tunnel incision or a self sealing scleral tunnel incision. The width of the incision may vary between 1 and 4 mm.

In a further step (Fig. 14B) another instrument 110 is used, e.g. a dedicated folding forceps, to grasp a cover implant 10 and introduce it through the incision Inc.

The forceps include a pair of arms 112, 114 that flank the cover implant 10 along its longitudinal edges (supporting members 24, 26). In particular, arms 112, 114 are engaged into the respective first receiving portions 24a, 26a (the latter have shapes that are adapted to that of the arms, i.e. a semi-cylindrical shape to house a cylindrical or substantially cylindrical shape) to carry the cover implant 10.

Alternatively, the cover implant 10 may be loaded into a dedicated cartridge injector and then inserted through the corneal or scleral tunnel incision into the anterior chamber of the eye.

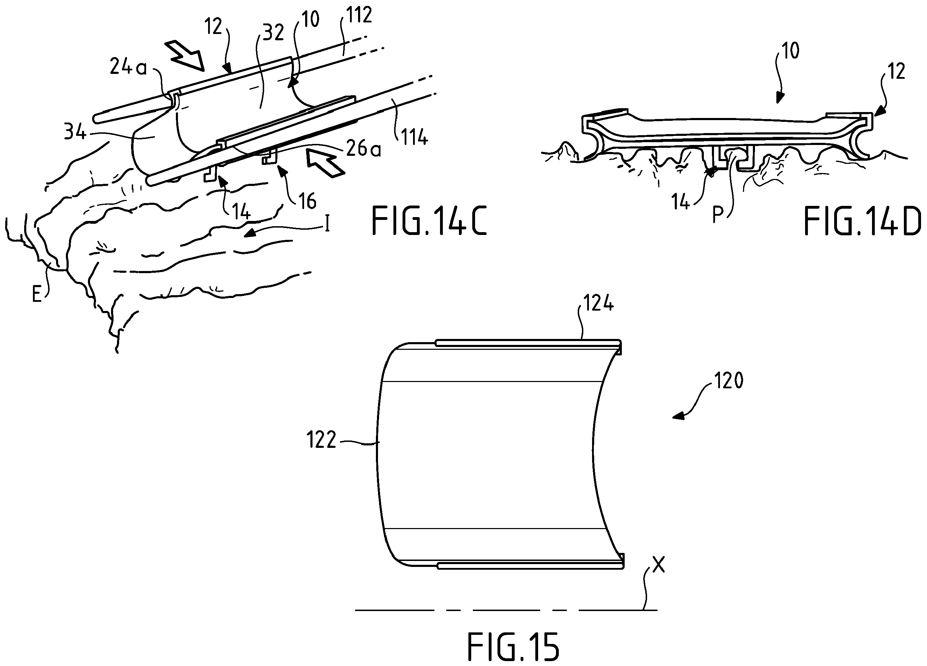

When the cover implant 10 is above the iris (Fig. 14B), at the required position and the forceps may be tightened so that the arms 112, 114 exert a clamping effort on the first receiving portions 24a, 26a of the cover implant as indicated by the facing arrows on enlarged Figure 14C. This clamping effort causes the cover implant to elastically bend as already explained with reference to Figures 5A-C. The attachment members 14, 16 are then caused to open above an iris radial ridge (natural fold of the iris surface) P to be clamped in a position adjacent to the pupil edge E.

Thus the cover implant 10 moves towards the radial ridge until the latter lies between the two clamping portions or jaws. Once correctly positioned on either part of the ridge P, the clamping effort can stop so that the two clamping portions or jaws clamp the ridge as illustrated by Figure 14D as a consequence of elastic return of the cover implant frame. The cover implant is therefore released from the instrument and positioned as illustrated in Figure 11A with the frame 12 and mechanical portion 32 above the iris and the cantilevered opaque optical portion 34 above the pupil aperture O1.

The same process is repeated (from Fig. 14B) as many times as the number of cover implants 10 to be implanted.

To be noted that radial and longitudinal positioning of the cover implant may be guided by means of a dedicated corneal marker impregnated with sterile biocompatible ink to indicate the central optical clear zone (minimum diaphragm diameter) and any adequate number of equally spaced radii or by any other optical means including the projection of a pattern of visible laser light through the cornea onto the iris surface.

More generally, the surgeon may use a positioning assist system, including inter alia an optical guide target (e.g. a laser with a visible wavelength) that will represents the optimal position of the implants on the iris, through the cornea.

The method may be performed with the pupil in its more constricted position so as to optimize implants positioning in order to have in this position an artificial diaphragm with a minimum diameter and with relative positioning of the implants that ensures no mechanical blocking.

Generally speaking, the opaque optical portion (or at least the opaque optical face of the cover member) of the iris cover implant may be designed according to various geometrical shapes (see for example Figs 10A-E, 13A-D and Figs 16-17) in order to optimize the optical continuity of the dynamic diaphragm effect when pupil diameter changes with light adaptation or accommodation. The shape of the opaque portion (or at least the opaque optical face of the cover member) may include additional oversized flange that may be tilted to create an overlapping effect with adjacent cover implants.

Figures 15 to 18 illustrate other applications for a new iris cover implant according to the invention.

Figure 15 illustrates an embodiment of an iris cover implant 120 that is intended to partially cover the iris only (no optical diaphragm function is present here).

Cover implant 120 comprises a cover member 122 corresponding to cover member 32 of Figure 4A and a frame 124 corresponding to frame 12 of Figures 1-3 and which is assembled with cover member 122, e.g. as previously described. Cover member 122 has an opaque face (upper face visible from a top view; the attachment members are located on an opposite face of the frame) and may be entirely opaque. Cover implant 120 may alternatively be made in a single piece.

The axial extension (along axis X) of cover member 122, and possibly frame 124, may be lengthened so as to cover a greater area of the iris anterior surface.

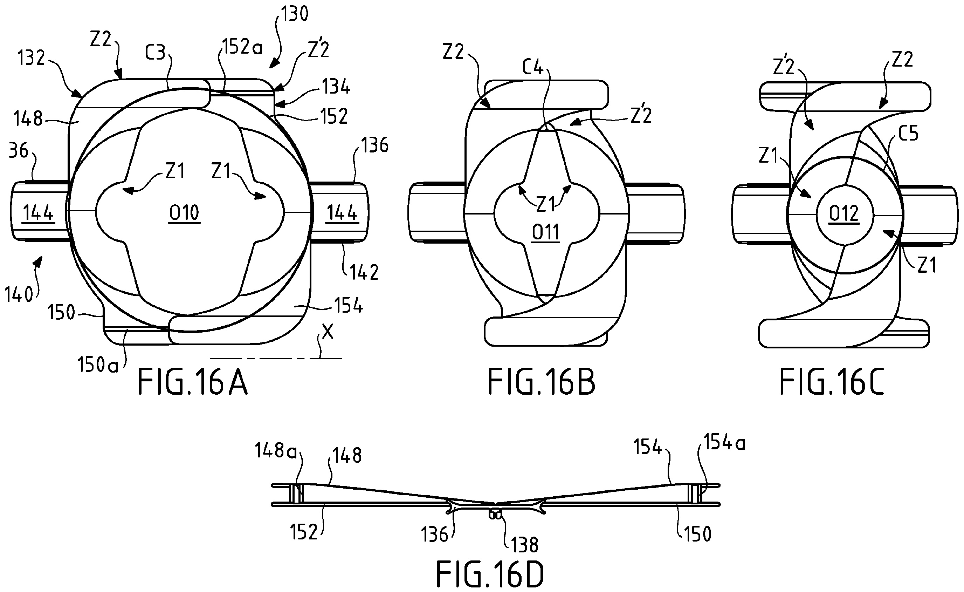

Figures 16A-D illustrate an embodiment of an assembly of several cover implants 132, 134 (here two facing segments are represented, e.g. having each a U shape, but more than two segments may be used in an alternative embodiment) forming a single intraocular implant which here acts both as a diaphragm and a partial artificial iris (iris cover). The partial iris cover may cover/overlap local iris deformities etc.

The two cover implants 132, 134 are assembled with each other through a sliding arrangement. However, other alternative assembling arrangements may be envisaged.

Each cover implant comprises a body having a frame 136 with attachment members 138 (Fig. 16D) as frame 12 in Figures 1-3 and a cover member 140, 142. Cover member 140 has an opaque face (upper face visible from a top view; the attachment members are located on an opposite face of the frame) and may be wholly opaque.

Each cover member 140, 142 has two portions:
- a main identical portion 144 as mechanical portion 32 in Figure 4A;
- a secondary portion that has two laterally extending arms 148, 150 and 152, 154 both connected to main portion 144.

The two arms of the two cover members are in register with each other.

One of the two arms lies substantially in a plane as that of Figures 16A-C (planar configuration) whereas the other arm is in a raised position relative to the first arm and at an angle with the latter.

Figure 16D shows the inclined raised arms 148, 154 of the two facing cover members that are mechanically engaged into each other to form an assembly.

This arrangement makes it possible for each raised arm of a cover member to overlap, to a small or greater extent, the corresponding planar arm of the other cover member (see Figs. 16A-C).

The raised arms are each provided with a protruding member, e.g. a lug, a rib etc., 148a, 154a (Fig. 16D) and the planar arms are each provided with a longitudinal slot 150a, 152a (Fig. 16A) in which the protruding member is able to axially slide (along axis X) so that the two cover members can move relative to each other as in Figures 16A-C. Here the protruding members are downwardly extending from the lower face of the arms.

The two arms of each cover member comprise each a first zone Z1 corresponding to an opaque optical zone that is intended to play the role of a diaphragm (this is an optical portion with an optical face).

The two arms of each cover member also comprise each a second zone Z2 and Z2' corresponding to an opaque non optical zone. Zone Z2, Z2' has two spaced apart portions that flank the first central zone Z1.

The above described sliding arrangement is carried by second zone Z2, Z2', in particular, by the two spaced apart portions that flank the first central zone Z1.

In Figure 16A, a circle C3 identifies the natural dilated pupil with a central aperture O10. In this position the two zones Z1 extend above aperture O10 and zones Z2 and Z2' partially extend above aperture O10.

In Figures 16B and 16C, the circles C4, C5 identify respectively more and more constricted pupil positions. In these positions the two zones Z1 extend above reduced apertures O11 and 012 respectively whereas zones Z2 and Z2' do no longer overlap these apertures. The two zones Z1 act as a diaphragm, in particular in the fully constricted position of Figure 16C. The zones Z2 and Z2' act as an artificial iris and cover a part of the natural iris.

In the present embodiment the main portions 144 and the zones Z2 and Z2' have each a transparent face or an opaque one (pigmented, textured and/or colored or not) so as to cover the iris either for locally masking a deformity, etc. and/or for aesthetic reasons (color change etc.).

The zones Z1 have both an opaque optical face (the whole zones in their entirety may be opaque), e.g. a black face to play the role of a diaphragm when moved close to each other as in Figure 16C.

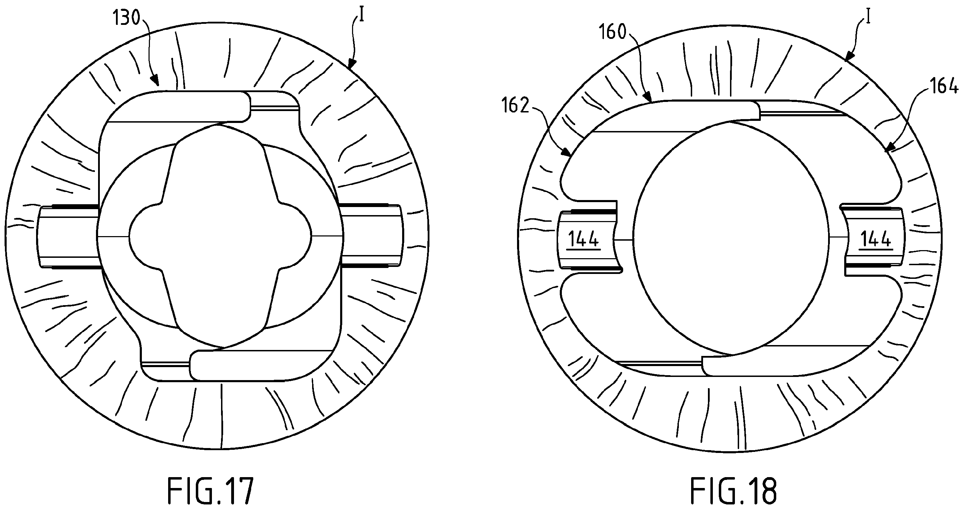

Figure 17 illustrates the assembly 130 of Figures 16A-D attached to the anterior surface of iris I.

Other configurations may be envisaged depending on the anatomy of the patient's eye and his/her optical disorders. In particular, different shapes of iris cover implants, arms etc. may be envisaged to more or less cover the natural iris.

Figure 18 illustrates another assembly 160 of several iris cover implants
with no optical diaphragm function. Assembly 160 represents an artificial iris implant that covers at least partially the natural iris. Here the cover implants 162, 164 have a similar U shape as that of assembly 130 but, alternatively, may take any other shape. The two arms of each cover implant have a greater cover area than that of assembly 130 above the iris so as to overlap the latter to a greater extent. The optical zone of the arms extending beyond the iris edge has been removed here. The two arms of each cover implant extend laterally with respect to the main portion 144 of the cover member and rearwardly relative to main portion 144 so as to flank the latter on either side. The shape of the arms may vary according to the zone of the iris to be covered as for assembly 130 and may cover the whole iris (a few rules have to be taken into account for placing such an implant as will be seen subsequently) or not.

The features and advantages of assembly also apply here to assembly 160.

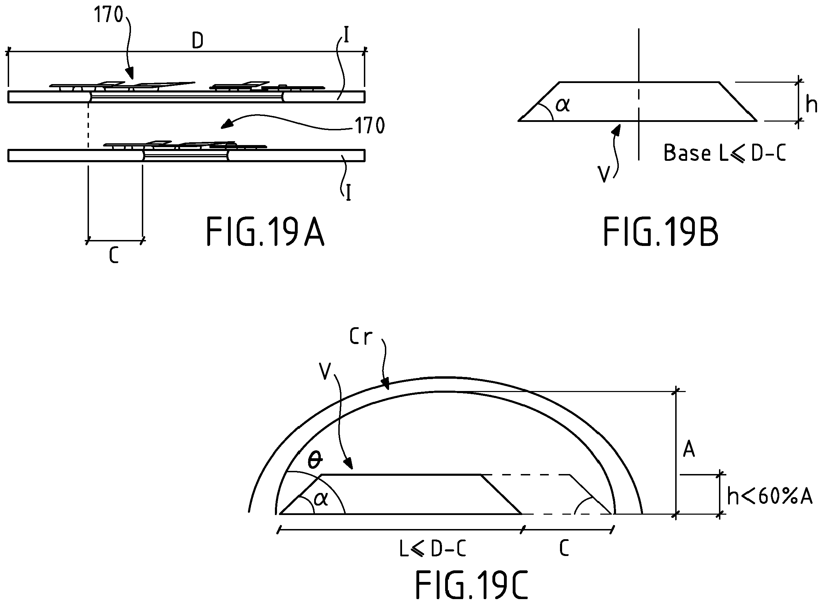

Figures 19A-C show the implantation of cover implants on the anterior surface of an iris I.

In Figure 19A, a set of several cover implants 170 are represented mounted onto the iris, around the pupil. Figure 19A shows an upper and a lower view: in the upper view the iris is in a dilated position whereas it is in a fully constricted position in the lower view. The whole external diameter of the iris (at its base) is referred to as D and C represents the radial displacement or travel of the iris between the two extreme positions. For example, the pupil diameter may be equal to 7 mm in the dilated position and to 3 mm in the fully constricted position, which leads to a 2 mm stroke for C.

Figure 19B represents a shape of a truncated cone with an angle α at its basis, a base with a width L that is less or equal than D-C and a height h.

This volume represents a typical volume V in which the cover implant(s) according to the invention may be installed, being understood that a security margin has to be taken so as to avoid placing an implant which would be in contact or too close to the iridocorneal angle (angle θ in Figure 19C).

Figure 19C illustrates the space that is authorized for installing an implant on the iris (the iris is not represented here for the sake of simplicity) given the size and shape of the cornea Cr.

The volume V has been represented in an offset position (this does not correspond to any actual design) to highlight the width C that is not authorized for implant installation.

Generally speaking, the angle α of the allowed volume is less than the angle θ, for instance less than 60% of this angle so as to avoid any contact between the implant(s) and the cornea Cr.

The length or width L is also preferable less than D-C for the same reasons.

The height h is less than the maximum axial distance A of the anterior chamber and for instance less than 60% of this distance for the same reasons as above.

Put it another way, the constraints that have to be taken into account before placing a cover implant on an iris depend on the actual diameter of the iris, the actual travel of the iris during its two extreme positions and the height of the cornea.

By way of example, for an average implant (i.e. an implant that is based on an average of biological data measurements obtained on a plurality of patients) its length or width L and height should not go beyond 7.5 mm and 1.5 mm for space physiological reasons. Anyway, the size of the implant is to be made as small as possible.

## Claims

1. An iris cover implant intended to cover at least partially the iris of an eye, the cover implant comprising a body having a first face that is an opaque face and, on an opposite second face, at least one attachment member that extends outwardly from the body, the at least one attachment member being able to attach the body to an iris of an eye.

2. The cover implant according to claim 1, **characterized in that** the opaque face extends at least in a plane that is substantially perpendicular to the direction of extension of the at least one attachment member relative to the second face of the body.

3. The cover implant according to claim 1 or 2, **characterized in that** the at least one attachment member comprises at least two portions that are able to move away from each other under the action of an external force in a plane containing the direction of extension of the at least one attachment member, the body being able to elastically bend within the plane when submitted to an external force so as to cause said at least two portions to move away from each other from an initial position, said at least two portions being able to return to their initial position in the absence of any external force.

4. The cover implant according to claim 3, **characterized in that** the body has at least two receiving portions that are each adapted to receive an instrument or a portion of an instrument for elastically bending the body.

5. The cover implant according to any of claims 1 to 4, **characterized in that** the body has at least two attachment members spaced apart from each other along a first direction that is substantially perpendicular to the direction of extension of the at least one attachment member relative to the second face of the body, the body having a thickness along the direction of extension of the at least one attachment member relative to the second face of the body , the thickness of the body being reduced in a zone of the body that is located between the two zones where the at least two attachment members are disposed.

6. The cover implant according to any of claims 1 to 5, **characterized in that** the body comprises a frame provided with the at least one attachment member and a cover member assembled with the frame, the cover member including the opaque face.

7. The cover implant according to claim 6, **characterized in that** the frame and the cover member are mechanically engaged with each other.

8. The cover implant according to claim 7, **characterized in that** the frame has second receiving portions that are each adapted to receive the cover member.

9. The cover implant according to claim 5 and any of claims 6 to 8, **characterized in that** the frame comprises two parallel spaced apart beams with two attachment members located on the two beams respectively.

10. The cover implant according to any of claims 6 to 9, **characterized in that** the cover member also includes a mechanical portion that is assembled with the frame.

11. The cover implant according to any of claims 6 to 10, **characterized in that** the cover member and the frame are each able to be elastically deformed.

12. The cover implant according to any of claims 1 to 11, **characterized in that** the body comprises a frame provided with the at least one attachment member and a cover member including the opaque face, the frame and the cover member being made of a single piece.

13. The cover implant according to any of claims 1 to 12, **characterized in that** the opaque face has at least one zone that is an opaque optical face.

14. The cover implant according to any of claims 6 to 12 and according to claim 13, **characterized in that** the opaque optical face belongs to an optical portion that extends beyond the frame.

15. A surgical kit comprising a plurality of cover implants as in any of claims 1 to 14, the plurality of cover implants forming a multi-component iris cover implant acting as a diaphragm when attached to an iris of an eye.

16. A surgical kit comprising a plurality of cover implants as in any of claims 1 to 14, the plurality of cover implants forming a multi-component iris cover implant acting as a partial or total artificial iris when attached to an iris of an eye.

17. An assembly of several cover implants as in any of claims 1 to 14, the plurality of cover implants forming a single iris cover implant acting as a diaphragm when attached to an iris of an eye.

18. An assembly of several cover implants as in any of claims 1 to 14, the plurality of cover implants forming a single iris cover implant acting as a partial or total artificial iris when attached to an iris of an eye.
